# EUROPEAN PATENT APPLICATION

(11) **EP 1 571 212 A1**
(43) Date of publication of application: **07.09.2005**
(21) Application number: 05004542.6
(22) Date of filing: 02.03.2005
(51) Int. Cl.: C12N 15/12, C07K 14/765, A61K 38/38

(54) **Recombinat protein containing albumin multimer**

(30) Priority: 03.03.2004 JP 2004058620
(71) Applicant: NIPRO CORPORATION, 531-8510 (JP)
(72) Inventor: Otagiri, Masaki, Kumamoto-shi, Kumamoto-ken 862-0938 (JP); Kida, Yoshinori, c/o Nipro Corporation, Osaka-shi, Osaka-fu 531-8510 (JP); Katayama, Naohisa, c/o Nipro Corporation, Osaka-shi, Osaka-fu 531-8510 (JP); Kai, Toshiya, c/o Nipro Corporation, Osaka-shi, Osaka-fu 531-8510 (JP)
(74) Representative: Hiebl, Inge Elisabeth

(57) **Abstract**

A recombinant protein containing a serum albumin multimer wherein at least two genes encoding serum albumin are combined with each other by a gene recombinant technology and allow the production of a serum albumin protein in the host cell is provided. The gene encoding a human serum albumin multimer and the recombinant vector are also provided. The protein is used as a safe drug administration carrier that is capable of extending the half life of a drug in the blood without the risk of contamination of viruses and so on, compared with the conventional drug administration carrier.

## Description

### BACKGROUND OF THE INVENTION

The present invention relates to a protein containing a multimeric form of serum albumin which is produced by recombinant technology. More particularly, the present invention relates to a protein containing a multimeric form of serum albumin which is produced by recombinant technology and can be used as a drug administration carrier or the like with increased drug retaining property while preventing the risk of contamination with viruses and so on.

### BACKGROUNG ART

Human serum albumin (HSA) is a main protein found in the serum of an adult, is produced in the liver, and has a function as a carrier for transporting various serum molecules in a human body. In addition, the albumin has an important role in maintaining at a normal level a plasma colloid osmotic pressure caused by a solute (colloid) which cannot pass through pores of a capillary vessel to maintain a liquid content in blood. Therefore, the albumin has been administered for various treatments of surgery, mental shock, burn, and a hypoproteinemia that causes an edema, which are associated with a liquid loss from a blood vessel.

Serum albumin is the most abundant protein in the plasma and the average level thereof reaches 48 mg/mL (0. 67 mmol/L). There are two reasons for the abundance of albumin. Firstly, albumin is enriched such that the transcription of mRNA for an albumin precursor is facilitated in the liver cells by a tissue-specific mechanism. Secondly, the elimination and metabolism of albumin after being released into the blood circulation are comparatively slow. The metabolic half life of albumin is approximately 19 days in a human being and 4.6 to 6.2 days in a rabbit.

In this way, high retention of serum albumin in the blood has been known. Using the serum albumin as a drug administration carrier may improve the retention of a drug in the blood.

Meanwhile, pharmaceutical preparations containing albumin derived from the blood may be contaminated with an unknown virus. Thus, the use thereof to a human body or the like has caused safety problems. However, a method of producing serum albumin by using a microorganism transformed by a recombinant technology has been already proposed (see JP S58-56684 A and JP H05-292993 A), and thus, a safe serum albumin preparation may be provided.

Albumin is a protein having a single strand structure consisting of 585 amino acids and composed of three homologous domains. It has been known that albumin is acceleratedly secreted from the kidney when a native albumin having three domains is cleaved into proteins having one or two domains. In contrast, it has been known that an administration of a rabbit serum albumin dimer having six domains to a rabbit accelerates the disappearance of native albumin in the plasma (see McCurdy TR et al. , Journal of Clinical Medicine, 143(2). 115-124, 2004 February).

### SUMMARY OF THE INVENTION

An object of the present invention is to provide a safe drug administration carrier that is capable of extending the half life of a drug in the blood without fear of contamination of viruses, compared with the conventional drug administration carrier.

For solving the above problems, the inventors of the present invention have established a system for expressing a protein containing a serum albumin multimer by a recombinant technology and found out that the protein containing the serum albumin multimer has higher retention in the blood than that of a serum albumin monomer, thereby achieving the present invention.

That is, the present invention relates to;
(1) a protein containing a multimeric form of serum albumin which is produced by a recombinant technology;
(2) a protein according to the above item (1), in which the serum albumin has an amino acid sequence same as a human serum albumin;
(3) a pharmaceutical preparation containing the protein according to the above item (1);
(4) a DNA fragment containing at least two DNA sequences encoding a serum albumin;
(5) a DNA fragment according to the above item (4), further containing at least one restriction enzyme cleavage site between DNA sequences encoding a serum albumin;
(6) a recombinant vector containing the DNA fragment according to the above item (4);
(7) a host cell transformed with the recombinant vector according to the above item (6);
(8) a method of producing the protein according to above item (1), characterized by comprising; incorporating a DNA fragment containing at least two DNA sequences encoding a serum albumin into a vector; transforming a host cell with the vector; incubating a resulting transformant to produce a protein; and collecting the protein produced; and
(9) a method of producing the DNA fragment according to the above item (4) characterized by comprising; introducing a DNA fragment in which a restriction enzyme cleavage site is intervened between multiple DNA sequences encoding serum albumin, into a host cell having high proliferation potency; incubating a transformant obtained; extracting DNA from proliferated cells; and cleaving the DNA with a specific restriction enzyme.

The protein of the present invention has a prolonged half life in the body owing to a decrease in an elimination rate in the blood, so that the retention of a drug in the blood can be increased when the protein is used as a carrier for administrating the drug. In addition, as the protein is produced using a recombinant technology, there is no risk of contamination of unknown viruses and so on, an inherent problem in blood preparations. Therefore, the protein can be used safely for the human body and so on. Besides, the protein is composed of a component inherently present in the living body. Therefore, there is a little influence such as a side effect even if the protein is administered to the human body.

### Brief Description of the Drawings

Fig. 1 is an explanation diagram of illustrating general procedures for the production of a human serum albumin dimer of Example 1.
Fig. 2 is a schematic diagram of pKF18K-HSA.
Fig. 3 is a schematic diagram of a DNA fragment W-1 in Example 1.
Fig. 4 is a schematic diagram of a DNA fragment W-2 in Example 2.
Fig. 5 is a graphical representation of a time elapsed from the administration and a residual rate to a given dosage with respect to the protein of the present invention in Test Example 1.
Fig 6 is a schematic diagram of pPIC9K-HSA dimer in Example 2.
Fig. 7 is an explanation diagram of illustrating a procedure for the recombination of a Pichia genome in Example 2 in which HSA dimer cDNA is contained.
Fig. 8 shows sense and antisense primers used for amplifing a part of pPIC9K, HSA cDNA-1 and HSA cDNA-2 in Example 2.

In the present invention, the multimeric form of the serum albumin means one protein in which plural serum albumins are directly or indirectly coupled together. The multimeric form of the serum albumin is occasionally called a serum albumin multimer or a fusion protein. The serum albumin is generally human serum albumin. The serum albumin comprises a protein with a single strand structure consisting of 585 amino acids and composed of three homologous domains, the fragments of the protein and the modified protein or the fragment. The serum albumin multimer can be produced using a recombinant technology. The multimer includes a dimer or a trimer.

In the present invention, the DNA sequence encoding serum albumin is capable of generating serum albumin in a system that allows the expression of a gene containing the DNA sequence. The DNA sequence is not limited to a DNA sequence that generates a complete serum albumin protein and includes a DNA sequence that generates a partial polypeptide of the serum albumin. Preferably, however, the DNA sequence is the DNA sequence coding the complete serum albumin. In addition, the DNA sequence coding the serum albumin multimer preferably does not contain any intron homologous to mRNA encoding the whole or part of the serum albumin between two cDNA sequences encoding the serum albumin.

A DNA fragment that contains at least two DNA sequences encoding a serum albumin is a DNA fragment in which the above DNA sequence is present in a repetitive manner in the whole sequence of the DNA fragment. The plural DNA sequences in the DNA fragment may be identical to or different from each other. The DNA fragment means an isolated fragment.

Furthermore, a DNA fragment having at least one restriction enzyme cleavage site is preferably inserted between two DNA sequences encoding the serum albumin. The DNA fragment containing at least one restriction enzyme cleavage site as well as the DNA sequence encoding serum albumin multimer is ligated with a vector and the recombinant vector can be incorporated into a host cell having excellent proliferative capacity, such as E.coli to transform the host cell, and the transformant is then incubated. Subsequently, DNA (a plasmid) is extracted from the proliferated transformant and then cleaved with a restriction enzyme*¹, followed by purifying the DNA fragment. Consequently, a large amount of copies of the DNA fragment encoding the albumin multimer can be efficiently obtained. Besides, the copies of the DNA fragment can be actually used to make the recombinant vector and incorporated into a host cell for the production of the protein containing the serum albumin multimer effectively.
* 1: The restriction enzyme is different from the restriction enzyme which is used to ligate the albumin monomer.

Each of various known methods can be used as a method of incorporating the above DNA fragment into a vector. For instance, there is used a method involving adding ligase to a mixture solution of the DNA fragment treated with various restriction enzymes and the vector to combine the DNA fragment with the vector. The vector used may be any vector employed in a recombinant technology, and a plasmid vector is generally used.

Subsequently, the above recombinant vector is introduced into a host cell to obtain a transformant. A method of introducing a recombinant vector into the host cell may be any of the methods conventionally used in the art including a competent cell method, a protoplast method, a calcium phosphate co-precipitation method, an electroporation method, a microinjection method, a liposome fusion method, and a particle gun method. An arbitrary method may be applied depending on the host used.

The host cells used are preferably eukaryotic cells. Examples of the eukaryotic cells include *Phichia pastoris, Saccharomyces cerevisiae*, and *Shizo Saccharomyces pombe. Phichia pastoris* is preferred.

The thus-obtained transformant is incubated, and a fusion protein containing the serum albumin multimer is then produced in a culture. The protein is isolated by means of a known method and optionally purified to obtain the protein of the present invention.

A medium for incubating the transformant is one of the known media including: a nutrient medium such as a YPD medium; a minimal medium such as an MB medium; a BMMY medium; and a BMGY medium. The transformant is incubated at generally about 16 to 46°C, preferably about 25 to 37°C, for about 8 to 168 hours, preferably about 24 to 120 hours. The transformant may be incubated by a shaking or stationary culture, or additionally with stirring and aeration if necessary.

Examples of a known method of isolating and purifying a fusion protein produced in the culture include: a method using a difference in solubility such as salt precipitation or solvent precipitation; a method using a difference in molecular weight such asdialysis,ultrafiltration,or gel-electrophoresis;amethod using a difference in charge such as ion-exchange chromatography; a method using specific affinity such as affinity chromatography; a method using a difference in hydrophobicity such as reversed-phase high performance liquid chromatography; and a method using a difference in isoelectric point such as isoelectric focusing.

Examples of a known method of identifying an isolated and purified fusion protein include a western blotting method and an activity measurement method. In addition, the purified fusion protein can be subjected to an amino acid analysis, an amino terminal analysis, a primary structure analysis, or the like, to thereby clarify its structure.

### Example 1

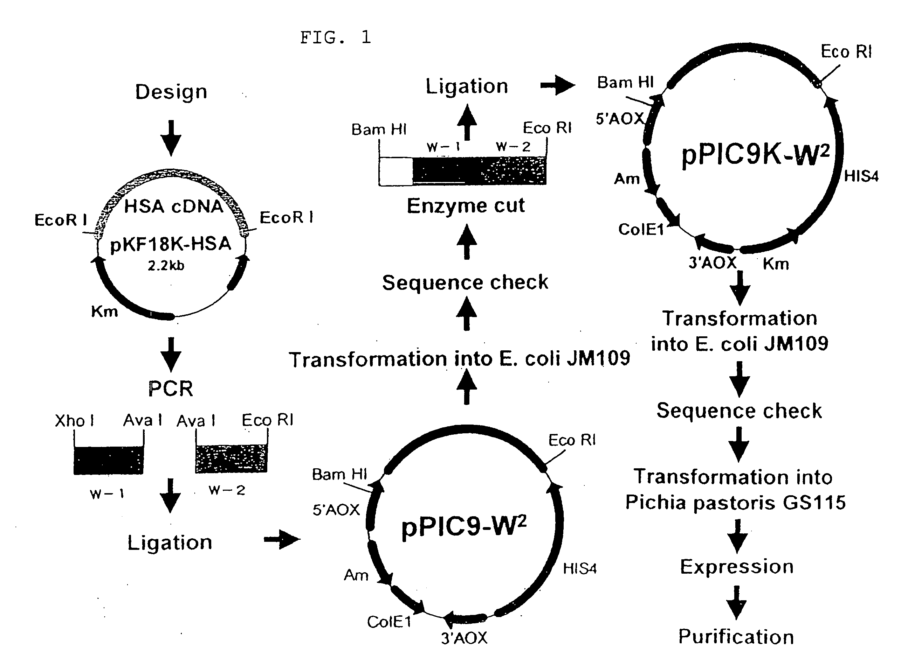

Fig. 1 shows an outline of procedures of Example 1.

### (Amplification of human serum albumin gene)

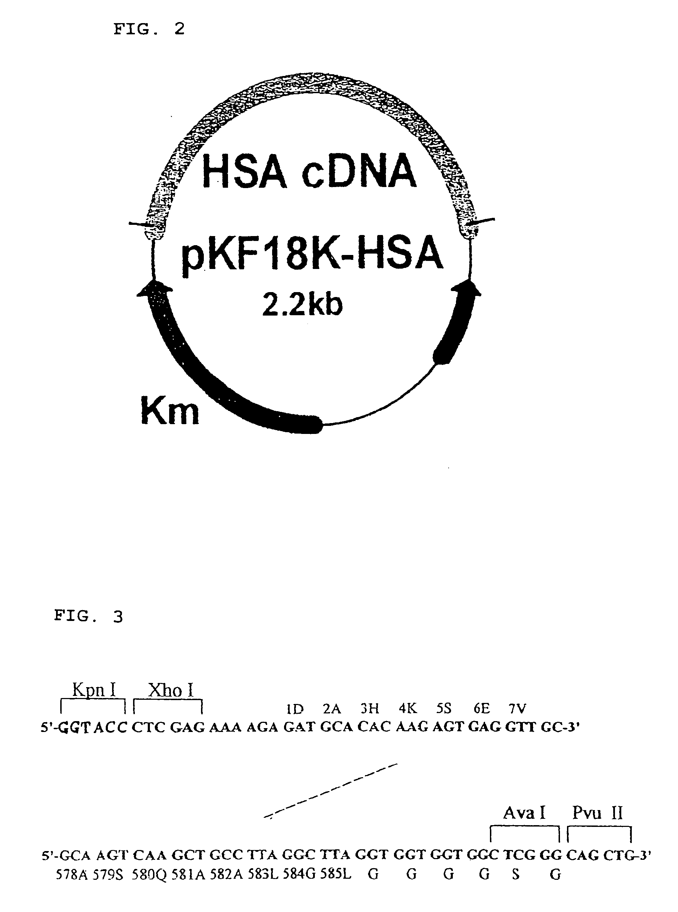

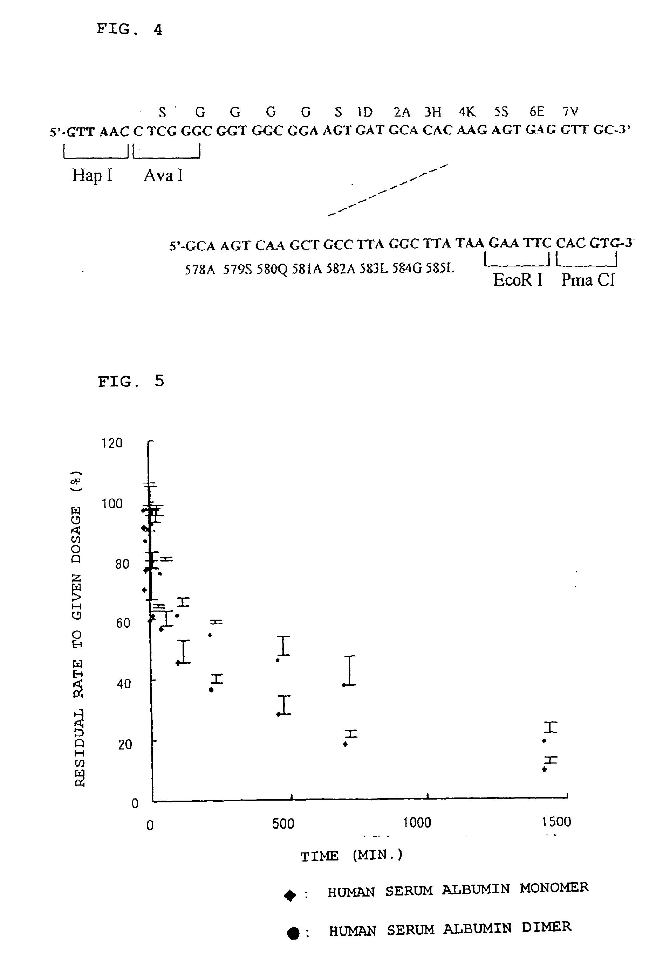

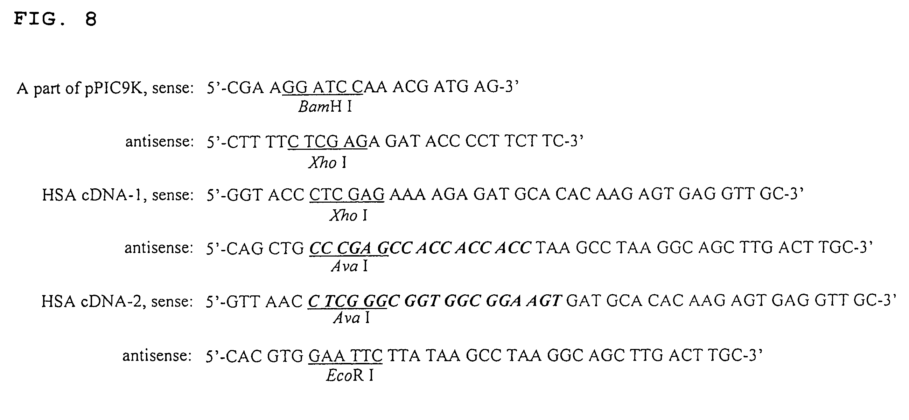

A plasmid prepared by incorporating a human serum albumin gene into a plasmid pKF18K (hereinafter, pKF18K-HSA, available from TonenGeneral Sekiyu K.K.) (see Fig. 2) was used as a template. A W-1 sense primer of SEQ. ID. No. 1 and a W-1 anti-sense primer of SEQ. ID. No. 2, and a W-2 sense primer of SEQ. ID. No. 3 and a W-2 anti-sense primer of SEQ. ID. No. 4 (shown in Fig. 8) were used as synthetic primers to carry out PCR using DNA polymerase (KOD-plus-DNA polymerase, available from Toyobo Co., Ltd.). As reaction conditions for PCR, DNA (a plasmid) was treated at 94°C for 2 minutes, subjected to a series of reactions of denaturing (94°C, 15 sec), annealing (63°C, 30 sec), and extension (68°C, 2 min) for 30 cycles, and then treated at 68°C for 5 minutes. DNA fragments, to which DNA sequences having restriction enzyme cleavage sites on the 3'-end and 5'-end portions of DNA sequences encoding human serum albumin were added, were amplified by the PCR. Therefore, a DNA fragment amplified by the sense primer of SEQ. ID. NO. 1 and the anti-sense primer of SEQ. ID. NO. 2 (hereinafter, W-1, see SEQ. ID. NO. 5 and Fig. 3), and a DNA fragment amplified by the sense primer of SEQ. ID. NO. 3 and the anti-sense primer of SEQ. ID. NO. 4 (hereinafter, W-2, see SEQ. ID. NO. 6 and Fig. 4) were obtained.

### (Linkage of Human Serum Albumin Gene)

The DNA fragment W-1 amplified by PCR was cleaved with the restriction enzyme Ava I (available from Takara Shuzo Co., Ltd.) after purification through phenol extraction and ethanol precipitation. On the other hand, the DNA fragment W-2 was cleaved with the restriction enzyme Ava I (available from Takara Shuzo Co., Ltd.) after purification through phenol extraction and ethanol precipitation. Subsequently, the DNA fragment W-2 was subjected again to the purification through phenol extraction and ethanol precipitation, followed by cleavage with the restriction enzyme Eco RI (available from Takara Shuzo Co., Ltd.). The DNA fragments W-1 and W-2, and a plasmid pPIC9 after restriction enzyme treatments were subjected to agarose gel electrophoresis. Then, the bands corresponding to the respective DNA fragments were cut out and then subjected to gel extraction using a gel extraction kit (QIAquick Gel Extraction Kit, manufactured by QIAGEN). After gel extraction, the DNA fragments W-1 and W-2, and the plasmid pPIC9 were mixed together and subjected to a ligation reaction at 16°C for 4 hours using a DNA ligation kit (DNA Ligation Kit Ver. 1, manufactured by Takara Shuzo, Co., Ltd.) to prepare a recombinant plasmid (hereinafter, referred to as pBIC9-W²) in which a DNA fragment encoding a serum albumin dimer (hereinafter, referred to as W²) was ligated with the plasmid pPIC9.

The pPIC9-W² thus obtained was introduced into *E. coli* JM109 to conduct transformation. The transformant introduced with the intended plasmid pPIC9-W² was screened in a medium supplemented with ampicillin and then the plasmid was purified using a plasmid purification kit (QIAprep Spin Miniprep Kit, manufactured by QIAGEN). The plasmid was subjected to double-cleavage with the restriction enzymes Xho I and Eco RI (available from Takara Shuzo Co., Ltd.) and then subjected to triple-cleavage with the restriction enzymes Xho I, Ava I, and Eco RI (available from Takara Shuzo Co., Ltd.) to prepare a restriction enzyme map, confirming that the plasmid was an intended plasmid vector.

The bacterium *E.coli* into which the intended plasmid had been introduced was confirmed and incubated. Then, from the proliferated bacterial cells, a plasmid (pPIC9-W²) was extracted and purified using a plasmid purification kit (QIAGEN plasmid Maxi Kit, manufactured by QIAGEN). The plasmid was subjected to double-cleavage with the restriction enzymes Bam HI and Eco RI (available from Takara Shuzo Co., Ltd.). After purification through phenol extraction and ethanol precipitation, agarose gel electrophoresis was conducted to cut out a band (3.5 kb) corresponding to W², followed by gel extraction using a DNA extraction kit (QIAquik Gel Extraction Kit, manufactured by QIAGEN).

### (Amplification of Human Serum Albumin Gene)

Likewise, subsequently, the DNA fragment W² was incorporated into the plasmid pPIC9K. The pPIC9K was cleaved with two restriction enzymes Bam HI and Eco RI (available from Takara Shuzo, Co., Ltd.) and then purified through phenol extraction and ethanol precipitation, followed by carrying out agarose gel electrophoresis and gel extraction with a gel extraction kit (QIAquik Gel Extraction Kit, manufactured by QIAGEN). The DNA fragment W² and the plasmid pPIC9K were mixed and subjected to a ligation reaction at 16°C for 4 hours using a DNA ligation kit (DNA Ligation Kit Ver. 1, manufactured by Takara Shuzo, Co., Ltd.) to prepare a plasmid in which the DNA fragment W² was ligated with the plasmid pPIC9K (hereinafter, referred to as pPIC9K-W²).

The plasmid pPIC9K-W² was introduced into *E.coli* JM109 to conduct transformation. The transformant was screened in a medium supplemented with kanamycin and then the plasmid was extracted and purified using a plasmid purification kit (QIAprep Spin Miniprep Kit, manufactured by QIAGEN), followed by confirming the introduction of the plasmid pPIC9K-W². The transformant into which the plasmid pPIC9K-W² was introduced had been confirmed and further incubated. From the proliferated bacterial cells, likewise, the plasmid was extracted and purified. Then, the plasmid was subjected to double-cleavage with the restriction enzymes Xho I and Eco RI (available from Takara Shuzo Co., Ltd.) and then subjected to triple-cleavage with the restriction enzymes Xho I, Ava I, and Eco RI (available from Takara Shuzo Co. , Ltd.) to prepare a restriction enzyme map. Simultaneously, using a DNA sequencer (ABI Prism 310 Genetic Analyzer, manufactured by Perkin-Elmer Applied Biosystems), the DNA sequence was identified to confirm the application of the intended DNA fragment W².

### (Expression of human serum albumin dimer)

The plasmid pPIC9K-W² was cleaved with the restriction enzyme Sal I and purified through phenol extraction and ethanol precipitation, followed by transformation through introduction of the plasmid pPIC9K-W² into *Pichia pastoris* GS115 by an electroporation method using an electroporation system (Gene Pulser II Electroporation System, manufactured by Bio-Rad Laboratories, Inc.). The resulting transformant was screened, and only positive clones exhibiting G418 resistance were incubated in a BMMY liquid medium. Then, the expression of an albumin protein was confirmed, and the transformant was then stored in glycerol.

### (Purification of human serum albumin dimer)

The transformed *Pichia pastoris* GS115 was incubated in a BMGY liquid medium for 48 hours and then incubated in a BMMY medium for 96 hours while 1% methanol was added every 12 hours. Yeast cells were isolated by means of centrifugation (6,000 g × 10 min), and a culture supernatant was then filtered through a 0.22-µm filter and then purified using a Blie affinity CL-6B column.

### (Test Example 1)

A human serum albumin dimer prepared by the same way as that of Example 1 was labeled with a radioactive indium isotope (¹¹¹In) to prepare an ¹¹¹In recombinant human serum albumin dimer. The ¹¹¹In recombinant human serum albumin dimer was intravenously administered to three laboratory mice through their caudal veins. After administration, the blood was sampled every constant time (at a certain time passed after adiministration) and the blood albumin level was determined by using a radiation dosage meter. As a control, ¹¹¹In human serum albumin prepared by labeling human serum albumin (monomer) with ¹¹¹In was administered to a mouse and then the same measurement was conducted. Fig. 5 shows the time elapsed from the administration and the residual rate to the given dosage.

As is evident from the results shown in Fig. 5, the human serum albumin diameter has a prolonged blood residence time, compared with the monomer. The half life of the former seems to be about five times as long as that of the latter.

The albumin of the present invention is a genetically produced protein originated from the natural protein and has no possibility of contamination with viruses and so on, so that it can be used as a non-toxic drug carrier. The albumin of the present invention can bind a large amount of drug in a molecule, so that it will be useful as a drug administration carrier capable of binding to a high molecular weight drug or the like.

### Example 2

### (Amplification of DNA sequence gene surrounding with recognition sites of Bam HI and Xho I in pPICK9)

A plasmid pPIC9K (pPIC9K) was used as a template to carry out PCR in the same manner as Example 1 for an amplification using a sense primer and an antisense primer shown in Fig. 8 (SEQ ID. 7 and 8). The amplified pPIC9K was purified by ethanol precipitation, double-digested by restriction enzymes Bam HI and Xho I (Takara Shuzo), and the digested fragments were applied to an electrophoresis, and then, a gel band corresponding to the DNA fragments having the DNA sequence surrounded by the recognition sites of BamHI and XhoI were cleaved out. A gel extracted DNA fragment (2) was obtained from the gel band by gel extraction kit (QIAquick Gel Extraction Kit, QIAGEN).

### (Ligation and amplification of the human albumin gene)

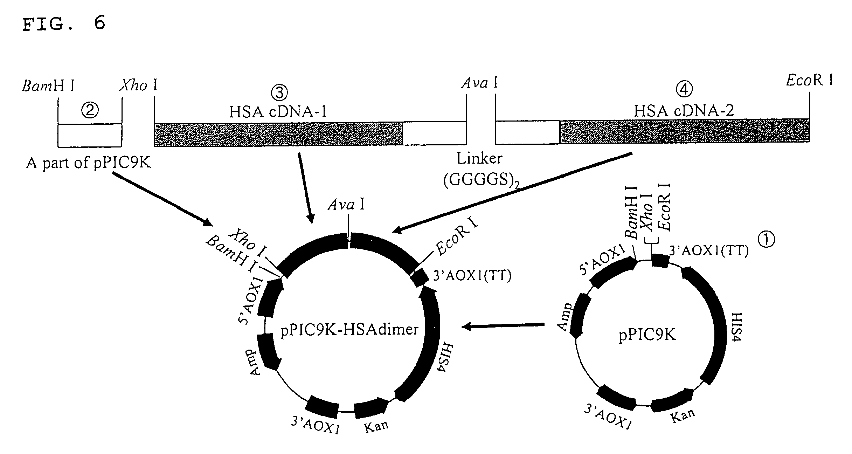

The DNA fragments W-1 (HSA cDNA-1, (3), Fig. 6) and W-2 (HSA cDNA-2, (4), Fig. 6) were gel extracted from pKF18K-HSA in the same manner as in Example 1. The sense and antisense primers for the DNA fragments W-1 and W-2 are shown in Fig. 8 (corresponding to SEQ. ID. No. 1 to 4). The DNA fragment (2), the fragments W-1 (3) and W-2 (4), and the plasmid, pPIK9 were ligated using DNA ligation kit (DNA Ligation Kit Ver.1, Takara Shuzo) for 4 hours at 16°C to make a plasmid, pPIC9K-HSA dimer (Fig. 6) in which the DNA fragments W-1 (3) and W-2 (4) are combined with the plasmid, pPIC9K.

The plasmid, pPIC9K-HSA dimer was transformed into a host cell E. coli JM109. The transformant was screened in a medium containing kanamycin, and the plasmids were extracted and purified by a plasmid purification kit (QIAprep Spin Miniprep kit, QIAGEN), and then, the plasmids were confirmed to contain the plasmid, pPIC9K-HSA dimer. The transformants which contains the plasmid, pPIC9K-HSA dimer were further incubated, and the plasmids were extracted and purified from the proliferated bacteria to double-digest with the restriction enzymes XhoI and EcoRI (Takara Shuzo), and subsequently triple-digested with restriction enzymes XhoI, AvaI and EcoRI (Takara Shuzo). The restriction mapping was made and simultaneously, the DNA fragments were confirmed by DNA sequencer (ABI Prism 310 Genetic Analyzer, Perkin-Elmer Applied Biosystem) that the intended DNA fragments was amplified therein.

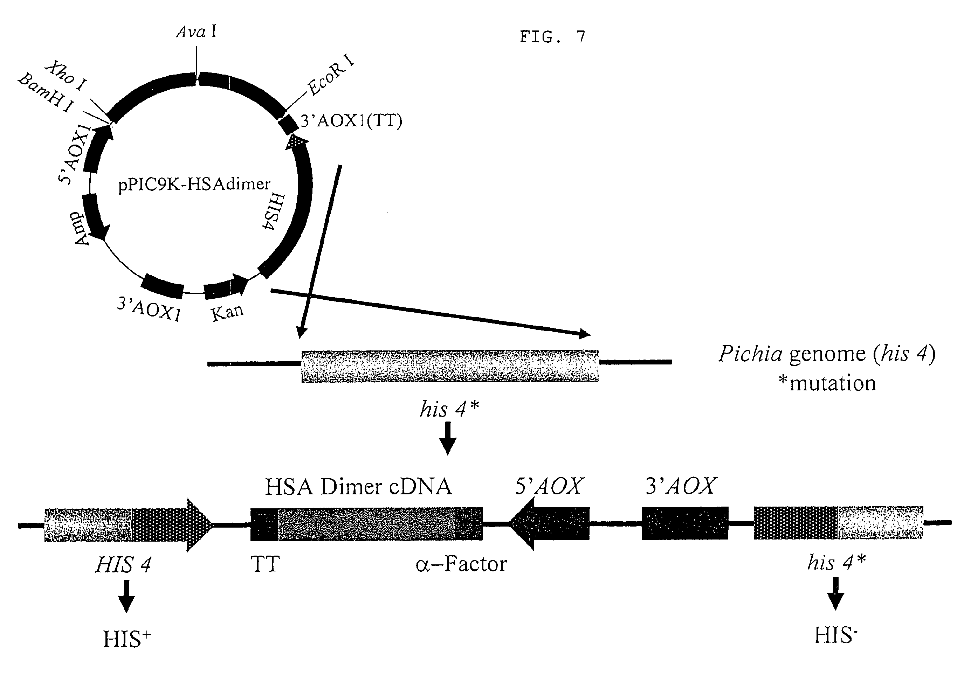

Subsequently, the plasmid pPIC9K-HSA dimer was introduced into Pichia GS115 genome (his 4) (see, Fig. 7) by recombination. The human serum albumin dimer was expressed in Pichia GS115, purified and isolated therefrom in the same manner as in Example 1.

## Claims

1. A protein comprising a human serum albumin multimer, which is produced by using a recombinant technology.

2. A protein according to claim 1, wherein the plural serum albumins are directly or indirectly coupled together.

3. A protein according to claim 1, wherein the serum albumin comprises a protein with a single strand structure consisting of 585 amino acids and composed of three homologous domains, the fragments of the protein and the modified protein or the fragment.

4. A protein according to claim 1, wherein the serum albumin comprises a protein with a single strand structure consisting of 585 amino acids and composed of three homologous domains.

5. A protein according to claim 1, wherein the multimer includes a dimer or a trimer.

6. A pharmaceutical preparation comprising the protein comprising a serum albumin multimer according to claim 1.

7. A DNA fragment comprising at least two DNA sequences encoding a serum albumin.

8. A DNA fragment according to claim 7, further comprising at least one restriction enzyme cleavage site between DNA sequences encoding a serum albumin.

9. A recombinant vector comprising the DNA fragment according to claim 7.

10. A host cell transformed with the recombinant vector according to claim 9.

11. A method of producing the protein according to claim 1, **characterized by** comprising incorporating a DNA fragment containing at least two DNA sequences encoding a serum albumin into a vector; transforming a host cell with the vector; incubating a resulting transformant to produce a protein; and collecting the protein produced.

12. A method of producing the DNA fragment according to claim 7, **characterized by** comprising; introducing a DNA fragment, in which a restriction enzyme cleavage site is intervened between two DNA sequences encoding serum albumin, into a host cell having high proliferation potency; incubating a transformant obtained; extracting DNA from proliferated cells; and cleaving the DNA with a restriction enzyme which has a different restriction site intervened between two DNA sequences encoding the serum albumin.


SEQUENCE LISTING

<110>  NIPRO CORPORATION

<120>  Recombinant protein containing albumin multimer

<130>  16-017

<160>  6

<170>  PatentIn version 3.1

<210>  1
<211>  40
<212>  DNA
<213>  Homo sapiens

<400>  1
ggtaccctcg agaaaagaga tgcacacaag agtgaggttg                              40

<210>  2
<211>  47
<212>  DNA
<213>  Homo sapiens

<400>  2
cagctgcccg agccaccacc acctaagcct aaggcagctt gacttgc                      47

<210>  3
<211>  48
<212>  DNA
<213>  Homo sapiens

<400>  3
gttaacctcg ggcggtggcg gaagtgatgc acacaagagt gaggttgc                     48

<210>  4
<211>  39
<212>  DNA
<213>  Homo sapiens

<400>  4
cacgtggaat tcttataagc ctaaggcagc ttgacttgc                               39

<210>  5
<211>  1796
<212>  DNA
<213>  Homo sapiens

<400>  5
ggtaccctcg agaaaagaga tgcacacaag agtgaggttg ctcatcggtt taaagatttg      60

ggagaagaaa atttcaaagc cttggtgttg attgcctttg ctcagtatct tcagcagtgt     120

ccatttgaag atcatgtaaa attagtgaat gaagtaactg aatttgcaaa aacatgtgta     180

gctgatgagt cagctgaaaa ttgtgacaaa tcacttcata ccctttttgg agacaaatta     240

tgcacagttg caactcttcg tgaaacctat ggtgaaatgg ctgactgctg tgcaaaacaa     300

gaacctgaga gaaatgaatg cttcttgcaa cacaaagatg acaacccaaa cctcccccga 360

ttggtgagac cagaggttga tgtgatgtgc actgcttttc atgacaatga agagacattt 420

ttgaaaaaat acttatatga aattgccaga agacatcctt acttttatgc cccggaactc 480

cttttctttg ctaaaaggta taaagctgct tttacagaat gttgccaagc tgctgataaa 540

gctgcctgcc tgttgccaaa gctcgatgaa cttcgggatg aagggaaggc ttcgtctgcc 600

aaacagagac tcaaatgtgc cagtctccaa aaatttggag aaagagcttt caaagcatgg 660

gcagtggctc gcctgagcca gagatttccc aaagctgagt ttgcagaagt ttccaagtta 720

gtgacagatc ttaccaaagt ccacacggaa tgctgccatg gagatctgct tgaatgtgct 780

gatgacaggg cggaccttgc caagtatatc tgtgaaaatc aggattcgat ctccagtaaa 840

ctgaaggaat gctgtgaaaa acctctgttg gaaaaatccc actgcattgc cgaagtggaa 900

aatgatgaga tgcctgctga cttgccttca ttagctgctg attttgttga aagtaaggat 960

gtttgcaaaa actatgctga ggcaaaggat gtcttcctgg gcatgttttt gtatgaatat 1020

gcaagaaggc atcctgatta ctctgtcgtg ctgctgctga gacttgccaa gacatatgaa 1080

accactctag agaagtgctg tgccgctgca gatcctcatg aatgctatgc caaagtgttc 1140

gatgaattta aacctcttgt ggaagagcct cagaatttaa tcaaacaaaa ctgtgagctt 1200

tttaagcagc ttggagagta caaattccag aatgcgctat tagttcgtta caccaagaaa 1260

gtaccccaag tgtcaactcc aactcttgta gaggtctcaa gaaacctagg aaaagtgggc 1320

agcaaatgtt gtaaacatcc tgaagcaaaa agaatgccct gtgcagaaga ctatctatcc 1380

gtggtcctga accagttatg tgtgttgcat gagaaaacgc cagtaagtga cagagtcaca 1440

aaatgctgca cagagtcctt ggtgaacagg cgaccatgct tttcagctct ggaagtcgat 1500

gaaacatacg ttcccaaaga gtttaatgct gaaacattca ccttccatgc agatatatgc 1560

acactttctg agaaggagag acaaatcaag aaacaaactg cacttgttga gcttgtgaaa 1620

cacaagccca aggcaacaaa agagcaactg aaagctgtta tggatgattt cgcagctttt 1680

gtagagaagt gctgcaaggc tgacgataag gagacctgct ttgccgagga gggtaaaaaa 1740

cttgttgctg caagtcaagc tgccttaggc ttaggtggtg gtggctcggg cagctg 1796

<210> 6
<211> 1795
<212> DNA
<213> Homo sapiens

<400> 6
gttaacctcg ggcggtggcg gaagtgatgc acacaagagt gaggttgctc atcggtttaa 60

agatttggga gaagaaaatt tcaaagcctt ggtgttgatt gcctttgctc agtatcttca 120

gcagtgtcca tttgaagatc atgtaaaatt agtgaatgaa gtaactgaat ttgcaaaaac 180

atgtgtagct gatgagtcag ctgaaaattg tgacaaatca cttcataccc tttttggaga 240

caaattatgc acagttgcaa ctcttcgtga aacctatggt gaaatggctg actgctgtgc 300

aaaacaagaa cctgagagaa atgaatgctt cttgcaacac aaagatgaca acccaaacct 360

cccccgattg gtgagaccag aggttgatgt gatgtgcact gcttttcatg acaatgaaga 420

gacatttttg aaaaaatact tatatgaaat tgccagaaga catccttact tttatgcccc 480

ggaactcctt ttctttgcta aaaggtataa agctgctttt acagaatgtt gccaagctgc 540

tgataaagct gcctgcctgt tgccaaagct cgatgaactt cgggatgaag ggaaggcttc 600

gtctgccaaa cagagactca aatgtgccag tctccaaaaa tttggagaaa gagctttcaa 660

agcatgggca gtggctcgcc tgagccagag atttcccaaa gctgagtttg cagaagtttc 720

caagttagtg acagatctta ccaaagtcca cacggaatgc tgccatggag atctgcttga 780

atgtgctgat gacagggcgg accttgccaa gtatatctgt gaaaatcagg attcgatctc 840

cagtaaactg aaggaatgct gtgaaaaacc tctgttggaa aaatcccact gcattgccga 900

agtggaaaat gatgagatgc ctgctgactt gccttcatta gctgctgatt ttgttgaaag 960

taaggatgtt tgcaaaaact atgctgaggc aaaggatgtc ttcctgggca tgtttttgta 1020

tgaatatgca agaaggcatc ctgattactc tgtcgtgctg ctgctgagac ttgccaagac 1080

atatgaaacc actctagaga agtgctgtgc cgctgcagat cctcatgaat gctatgccaa 1140

agtgttcgat gaatttaaac ctcttgtgga agagcctcag aatttaatca aacaaaactg 1200

tgagcttttt aagcagcttg gagagtacaa attccagaat gcgctattag ttcgttacac 1260

caagaaagta ccccaagtgt caactccaac tcttgtagag gtctcaagaa acctaggaaa 1320

agtgggcagc aaatgttgta aacatcctga agcaaaaaga atgccctgtg cagaagacta 1380

tctatccgtg gtcctgaacc agttatgtgt gttgcatgag aaaacgccag taagtgacag 1440

agtcacaaaa tgctgcacag agtccttggt gaacaggcga ccatgctttt cagctctgga 1500

agtcgatgaa acatacgttc ccaaagagtt taatgctgaa acattcacct tccatgcaga 1560

tatatgcaca ctttctgaga aggagagaca aatcaagaaa caaactgcac ttgttgagct 1620

tgtgaaacac aagcccaagg caacaaaaga gcaactgaaa gctgttatgg atgatttcgc 1680

agcttttgta gagaagtgct gcaaggctga cgataaggag acctgctttg ccgaggaggg 1740

taaaaaactt gttgctgcaa gtcaagctgc cttaggctta taagaattcc acgtg 1795

<210> 7
<211> 21
<212> DNA
<213> Homo sapiens

<400> 7
cgaaggatcc caaacgatga g 21

<210> 8

<211>  26
<212>  DNA
<213>  Homo sapiens

<400>  8
cttttctcga gagatacccc ttcttc                                          26

SEQUENCE LISTING

<110> Nipro Corporation

<120> Recombinant protein containing albumin multimer

<130> 15210EP

<140> EP05004542.6
<141> 2005-03-02

<150> JP2004-058620
<151> 2004-03-03

<160> 8

<170> PatentIn version 3.1

<210> 1
<211> 40
<212> DNA
<213> Artificial Sequence

<220>
<223> Primer
<400> 1
ggtaccctcg agaaaagaga tgcacacaag agtgaggttg 40

<210> 2
<211> 47
<212> DNA
<213> Artificial Sequence

<220>
<223> Primer
<400> 2

cagctgcccg agccaccacc acctaagcct aaggcagctt gacttgc 47

<210> 3

<211> 48

<212> DNA

<213> Artificial Sequence

<220>

<223> Primer

<400> 3
gttaacctcg ggcggtggcg gaagtgatgc acacaagagt gaggttgc 48

<210> 4

<211> 39

<212> DNA

<213> Artificial Sequence

<220>

<223> Primer

<400> 4
cacgtggaat tcttataagc ctaaggcagc ttgacttgc 39

<210> 5

<211> 1796

<212> DNA

<213> Artificial Sequence

<220>

<223> DNA fragment of human serum albumin gene

<400> 5
ggtaccctcg agaaaagaga tgcacacaag agtgaggttg ctcatcggtt taaagatttg 60

ggagaagaaa atttcaaagc cttggtgttg attgcctttg ctcagtatct tcagcagtgt 120

ccatttgaag atcatgtaaa attagtgaat gaagtaactg aatttgcaaa aacatgtgta 180

gctgatgagt cagctgaaaa ttgtgacaaa tcacttcata ccctttttgg agacaaatta 240

tgcacagttg caactcttcg tgaaacctat ggtgaaatgg ctgactgctg tgcaaaacaa 300

gaacctgaga gaaatgaatg cttcttgcaa cacaaagatg acaacccaaa cctcccccga 360

ttggtgagac cagaggttga tgtgatgtgc actgcttttc atgacaatga agagacattt 420

ttgaaaaaat acttatatga aattgccaga agacatcctt acttttatgc cccggaactc 480

ctttttcttg ctaaaaggta taaagctgct tttacagaat gttgccaagc tgctgataaa 540
gctgcctgcc tgttgccaaa gctcgatgaa cttcgggatg aagggaaggc ttcgtctgcc 600
aaacagagac tcaaatgtgc cagtctccaa aaatttggag aaagagcttt caaagcatgg 660
gcagtggctc gcctgagcca gagatttccc aaagctgagt ttgcagaagt ttccaagtta 720
gtgacagatc ttaccaaagt ccacacggaa tgctgccatg gagatctgct tgaatgtgct 780
gatgacaggg cggaccttgc caagtatatc tgtgaaaatc aggattcgat ctccagtaaa 840
ctgaaggaàt gctgtgaaaa acctctgttg gaaaaatccc actgcattgc cgaagtggaa 900
aatgatgaga tgcctgctga cttgccttca ttagctgctg attttgttga aagtaaggat 960
gtttgcaaaa actatgctga ggcaaaggat gtcttcctgg gcatgttttt gtatgaatat 1020
gcaagaaggc atcctgatta ctctgtcgtg ctgctgctga gacttgccaa gacatatgaa 1080
accactctag agaagtgctg tgccgctgca gatcctcatg aatgctatgc caaagtgttc 1140
gatgaattta aacctcttgt ggaagagcct cagaatttaa tcaaacaaaa ctgtgagctt 1200
tttaagcagc ttggagagta caaattccag aatgcgctat tagttcgtta caccaagaaa 1260
gtaccccaag tgtcaactcc aactcttgta gaggtctcaa gaaacctagg aaaagtgggc 1320
agcaaatgtt gtaaacatcc tgaagcaaaa agaatgccct gtgcagaaga ctatctatcc 1380
gtggtcctga accagttatg tgtgttgcat gagaaaacgc cagtaagtga cagagtcaca 1440
aaatgctgca cagagtcctt ggtgaacagg cgaccatgct tttcagctct ggaagtcgat 1500
gaaacatacg ttcccaaaga gtttaatgct gaaacattca ccttccatgc agatatatgc 1560
acactttctg agaaggagag acaaatcaag aaacaaactg cacttgttga gcttgtgaaa 1620
cacaagccca aggcaacaaa agagcaactg aaagctgtta tggatgattt cgcagctttt 1680
gtagagaagt gctgcaaggc tgacgataag gagacctgct ttgccgagga gggtaaaaaa 1740
cttgttgctg caagtcaagc tgccttaggc ttaggtggtg gtggctcggg cagctg 1796

<210> 6
<211> 1795
<212> DNA
<213> Artificial Sequence

<220>
<223> DNA fragment of human serum albumin gene
<400> 6
gttaacctcg ggcggtggcg gaagtgatgc acacaagagt gaggttgctc atcggtttaa 60
agatttggga gaagaaaatt tcaaagcctt ggtgttgatt gcctttgctc agtatcttca 120
gcagtgtcca tttgaagatc atgtaaaatt agtgaatgaa gtaactgaat ttgcaaaaac 180
atgtgtagct gatgagtcag ctgaaaattg tgacaaatca cttcataccc tttttggaga 240

caaattatgc acagttgcaa ctcttcgtga aacctatggt gaaatggctg actgctgtgc 300
aaaacaagaa cctgagagaa atgaatgctt cttgcaacac aaagatgaca acccaaacct 360
cccccgattg gtgagaccag aggttgatgt gatgtgcact gcttttcatg acaatgaaga 420
gacatttttg aaaaaatact tatatgaaat tgccagaaga catccttact tttatgcccc 480
ggaactcctt ttctttgcta aaaggtataa agctgctttt acagaatgtt gccaagctgc 540
tgataaagct gcctgcctgt tgccaaagct cgatgaactt cgggatgaag ggaaggcttc 600
gtctgccaaa cagagactca aatgtgccag tctccaaaaa tttggagaaa gagctttcaa 660
agcatgggca gtggctcgcc tgagccagag atttcccaaa gctgagtttg cagaagtttc 720
caagttagtg acagatctta ccaaagtcca cacggaatgc tgccatggag atctgcttga 780
atgtgctgat gacagggcgg accttgccaa gtatatctgt gaaaatcagg attcgatctc 840
cagtaaactg aaggaatgct gtgaaaaacc tctgttggaa aaatcccact gcattgccga 900
agtggaaaat gatgagatgc ctgctgactt gccttcatta gctgctgatt ttgttgaaag 960
taaggatgtt tgcaaaaact atgctgaggc aaaggatgtc ttcctgggca tgtttttgta 1020
tgaatatgca agaaggcatc ctgattactc tgtcgtgctg ctgctgagac ttgccaagac 1080
atatgaaacc actctagaga agtgctgtgc cgctgcagat cctcatgaat gctatgccaa 1140
agtgttcgat gaatttaaac ctcttgtgga agagcctcag aatttaatca aacaaaactg 1200
tgagcttttt aagcagcttg gagagtacaa attccagaat gcgctattag ttcgttacac 1260
caagaaagta ccccaagtgt caactccaac tcttgtagag gtctcaagaa acctaggaaa 1320
agtgggcagc aaatgttgta aacatcctga agcaaaaaga atgccctgtg cagaagacta 1380
tctatccgtg gtcctgaacc agttatgtgt gttgcatgag aaaacgccag taagtgacag 1440
agtcacaaaa tgctgcacag agtccttggt gaacaggcga ccatgctttt cagctctgga 1500
agtcgatgaa acatacgttc ccaaagagtt taatgctgaa acattcacct tccatgcaga 1560
tatatgcaca ctttctgaga aggagagaca aatcaagaaa caaactgcac ttgttgagct 1620
tgtgaaacac aagcccaagg caacaaaaga gcaactgaaa gctgttatgg atgatttcgc 1680
agcttttgta gagaagtgct gcaaggctga cgataaggag acctgctttg ccgaggaggg 1740
taaaaaactt gttgctgcaa gtcaagctgc cttaggctta taagaattcc acgtg 1795

<210> 7
<211> 21
<212> DNA
<213> Artificial Sequence

<220>
<223> Primer
<400> 7
cgaaggatcc caaacgatga g 21

<210>  8

<211>  26

<212>  DNA

<213>  Artificial Sequence

<220>

<223>  Primer

<400>  8
   cttttctcga gagatacccc ttcttc                                          26